# EUROPEAN PATENT SPECIFICATION

(11) **EP 2 762 053 B1**
(45) Date of publication and mention of the grant of the patent: **26.06.2019**
(21) Application number: 13153751.6
(22) Date of filing: 01.02.2013
(51) Int. Cl.: A47L 15/42

(54) **A method of operating a household appliance, and a household appliance, in particular a dishwasher**
Verfahren zum Betreiben eines Haushaltsgerätes und Haushaltsgerät, insbesondere ein Geschirrspüler
Procédé de fonctionnement d'un appareil ménager et appareil ménager, en particulier un lave-vaisselle

(43) Date of publication of application: 06.08.2014
(73) Proprietor: Rheinische Friedrich-Wilhelms-Universität Bonn, 53113 Bonn (DE)
(72) Inventor: Stamminger, Rainer, 53115 Bonn (DE); Boeker, Peter, 53225 Bonn (DE); Gilleßen, Claudia, 50374 Erftstadt (DE)
(74) Representative: Louis Pöhlau Lohrentz

(56) References cited:
- EP-A1- 2 196 576
- WO-A1-2005/047788
- WO-A1-2006/082552
- JP-A- H07 338
- JP-A- 2002 315 716
- JP-A- 2008 307 164
- US-A1- 2010 300 487

## Description

The invention relates to a method of operating a household appliance, in particular a dishwasher, in dependence on the presence of odours due to decomposition of organic material in that household appliance. The invention equally relates to a household appliance according to the preamble of claim 7, which is, e.g. known from JP 2008-307164 A.

In household appliances, deterioration of foodstuffs may occur and lead to arising of various odours. These odours depend on the food residues. Typically, such odours are unpleasant, and it is desired to avoid the occurrence of such odours. For example, if food residues may be present in a dishwasher, e.g. on dishes and cutlery, introduced some time ago, one foresees that the dishwasher becomes automatically active in a manner suitable to reduce odours due to these food residues. Typically in the dishwasher, an additional rinsing step takes place to remove the food residues from the dishes and cutlery.

It is also known to remove the odour itself.

In both cases, there must be a means triggering the action to reduce the odours.

US 2007/0131259 A1 discloses a method for eliminating odours in a dishwasher machine comprising a photocatalytic layer able to convert undesirable odours of organic origin at its surface into harmless substances. To that end, the photocatalytic layer needs to be exposed to UV-light, and air needs to circulate in the washing container. In one embodiment, this exposure of the photocatalytic layer and/or the circulation of the air located in the washing container are either sensor-controlled or time-controlled. In particular, it is disclosed that the sensor designed for this purpose could detect odours.

In JP 2008/307164 A, a dishwasher is disclosed which has two sensors. One sensor detects the smell of left-over food in the dishwasher. A second sensor detects the smell of detergent. The signals of both sensors determine whether or not an additional rinsing and addition of detergent takes place. If much left-over food is determined whilst there is not much detergent, the amount of detergent is increased.

It is extremely difficult to precisely measure all kinds of odours which can arise depending on food residues in household appliances. Usually, for precisely measuring odours, an individual sensor cannot be sufficient.

From the technical field of so-called electronic noses, it is known to use more than three and typically six to ten different sensors each responsive to different gases. If particular conditions are met such as the application of a reference gas, of provision of sample air, if the flux of the gases is controlled, and if humidity is stabilized, all of the sensors provide signals which are subjected to a pattern recognition.

It would be too extremely complicated to use six to ten sensors of an electronic nose and to provide for the conditions to be met in a household appliance such as a dishwasher.

The object of the present invention is thus to provide a method of operating a household appliance in dependence on the presence of odours due to decomposition of organic material in the household appliance as well as to provide a corresponding household appliance, wherein unpleasant odours are reliably detected and/or removed.

The object is solved by a method comprising the features of claim 1 and a household appliance comprising the features of claim 7.

The inventive method of operating a household appliance, in particular a dishwasher, in dependence on the presence of odours due to decomposition of organic material in said household appliance comprises, comparing measurement signals obtained by a first and a second sensor from which only the first sensor is responsive to at least one chemical substance which is produced upon decomposition of organic material, said comparing serving to determine whether a change in measurement values provided by the measurement signals is due to occurrence of a substance produced upon decomposition of organic material or due to a change in the environmental conditions.

The inventive method of operating a household appliance, in particular a dishwasher, in dependence on the presence of odours due to decomposition of organic material in said household appliance comprises the steps of:
- obtaining first measurement signals by a first sensor responsive (i.e. sensitive) to a first group comprised of at least one first chemical substance which is produced upon decomposition of organic material,
- obtaining second measurement signals by a second sensor responsive (i.e. sensitive) to a second group comprised of at least one second chemical substance which neither is produced upon decomposition of organic material, nor is a detergent or included in a detergent (which might be or is) used in said household appliance
- determining whether or not the value of a pre-determined function calculated both on the basis of the measurement values provided by the first measurement signals and the measurement values provided by the second measurement signals is higher than a pre-determined threshold value or whether or not the value of a pre-determined function calculated both on the basis of the measurement values provided by the first measurement signals and the measurement values provided by the second measurement signals is lower than a pre-determined threshold value, and if so,
   - outputting a signal indicating the presence of odours due to decomposition of organic material and/or a signal activating a unit in said household appliance acting to reduce such odours.

The invention is based on the principle to complement one sensor responsive to chemical substances occurring upon decomposition of organic material by a further sensor which is not responsive to such chemical substances: This further sensor helps in determining whether or not one is dealing with an effect on the measurement signals due to the occurrence of the chemical substances provided upon decomposition of organic material, or due to another effect (i.e. a change in the environmental conditions).

By this, it is avoided that due to the measurement signals of only one sensor, a cleaning step takes place even if that was not needed; for instance, some sensors for gases are very sensitive to the environmental conditions such that from the signals itself, one cannot unambiguously gather whether or not gases occur which are very odorous. If one makes use of two different sensors each sensitive to the environmental conditions in a comparable (according to a pre-determined criterion) manner, and if only one of the sensors is sensitive to the chemical substances provided upon decomposition of organic material, then, a "false alarm" can be detected: If the first sensor falsely "measures" odours when they are not present, the second sensor will equally provide for a comparable change in the measurement signals. The advantage is in particular provided when in the method, the physical, chemical or physico-chemical principles of how the first and the second measurement signals are obtained by the first and second sensors are (according to a pre-determined criterion) similar or are the same (i.e. identical).

It is to be noted that in the invention, if the value of the pre-determined function calculated both on the basis of measurement values provided by the first measurement signals and the measurement values
provided by the second measurement signals is higher than the pre-determined threshold value, the value of the inverse function will be lower than another pre-determined threshold value.

Preferably, the pre-determined function is a ratio of the measurement values provided by the first or the second measurement signals versus the measurement signal values provided by the second or first measurement signals, respectively. (The function calculated by the ratio of the first to the second measurement signals is the inverse of the ratio provided by the second to the first measurement signals).

Preferably, in all of the embodiments wherein a threshold value is used in a pre-determined criterion, and wherein in these embodiments a signal is output that activates a cleaning unit in the household appliance, it is foreseen that after the completion of a cleaning step, the measurement signals are obtained and stored for the determining of at least one threshold value used when applying at least one pre-determined criterion.

By this measure, one always obtains to the actual environmental condition the measurement signals in the situation wherein there are no odours (due to the learning step).

Preferably, the physical, chemical or physico-chemical principles of how the first and second measurement signals are obtained by the first and second sensors are identical. By this, there is one-to-one correspondence between a drift in the measurement signals output by the first sensors and a corresponding drift in the measurement signals output by the second sensor when there is a change in the environmental conditions. Only if there is a chemical substance occurring to which one of the sensors is sensitive and the other is not, only the measurement signals of the first sensor will undergo a change. Thus, one can unambiguously determine whether or not indeed these substances occur, and the influence of environmental conditions can be eliminated.

In a preferred embodiment, both sensors are metal oxide semiconductor sensors. Metal oxide semi-conductor sensors are well-known in the field and able to react/to be sensitive to a plurality of different gases. An overview on such sensors is given in the review of George F. Fine, Leon M. Cavanagh, Ayo Afonja and Russel Binions with the title "Metal Oxide Semi-Conductor Gas Sensors in Environmental Monitoring", in: Sensors 2010, 10, pages 5469-5502, ISSN 1424-8220. The principle of how these MOS sensors work includes both a physical and a chemical aspect, thus a physico-chemical principle. In order to apply a physical-chemical working principle for the two sensors which is as similar as possible, the second sensor should have as many similarities in its constitution as possible. In particular, one should apply the same process of manufacture for producing the two sensors, and use the same carrier (i.e. substrate). Most importantly, the same semiconductor oxide shall be used (with different dotation).

The measurement signals output by such sensors (abbreviated as "MOS sensors") always undergo a drift. If now, the first sensor is responsive to volatile organic compounds (which are produced upon decomposition of organic material), it is an indication of such volatile organic compounds when there is an increase in the measurement signals outputting the first sensor over a pre-determined time period. For that reason, preferably, a first pre-determined criterion is fulfilled when measurement values provided by the measurement signals increase over a pre-determined period of time in a pre-determined minimum amount (i.e. in at least a pre-determined amount).

It is to be noted that this first criterion, despite of monitoring the time development of the measurement signals, may be fulfilled both a) if at least one of the first chemical substances (the volatile organic compounds) is present in a minimum amount and b) if none of the first chemical substances is present in a respective minimum amount, but if another condition is met (such as, e.g., increase in temperature leading to an increase of the signal height).

In a further preferred embodiment, the second sensor is a equally a MOS sensor which is in particular responsive to hydrogene and/or nitrogene oxide(s) or any other substance which (extremely) rarely occurs in a household appliance and/or in the normal environment.

Hydrogene and nitrogene oxides do not occur upon decomposition of organic material. The second sensor such only serves to detect if the above-mentioned another condition is met (such as, e.g. increase in temperature leading to an increase of the signal height).

The inventive household appliance, in particular a dishwasher, comprises two sensors from which a first sensor is responsive (sensitive) to at least one first chemical substance which occurs when organic material is decomposing and a second sensor is responsive only to at least one second chemical substance which does not occur when organic material is decomposing, and this household appliance comprises a cleaning unit acting to remove sources of (unpleasant) odours in the household appliance, and further comprises a control unit receiving measurement signals from the first and second sensors and outputting control signals to said cleaning unit. The household appliance is characterised in that both the first and second sensors are responsive to the same environmental conditions and that said control unit is adapted to use signals of both the first and second sensors in the above described method when determining whether or not to output an activating control signal to said cleaning unit. By "responsive to environmental conditions", it is meant that if a parameter describing such environmental condition has a particular starting value and changes to an actual value, the output of the sensors changes equally, i.a. in a, according to a pre-determined criterion, comparable (similar) or even identical manner.

In other words, the household appliance according to the invention is configured/adapted to perform or make use of the inventive measurement of operating.

The preferred embodiments of the household appliance are indicated in the dependent claims. The advantages have been explained above.

In a further preferred embodiment, the control unit is adapted to continuously or repeatedly receive measurement signals from the first sensor and to determine whether or not a first pre-determined criterion is fulfilled. In case the pre-determined criterion is fulfilled, the control unit receives measurement signals from the second sensor and determines whether or not a second pre-determined criterion is fulfilled.

Here, the need is avoided to receive by the control unit measurement signals from both of the sensors all of the time, or at least to make use of both of such signals. There is only the need to observe the measurement signals from the first sensor. Only when the first pre-determined criterion is fulfilled, those from the second sensor are observed and made use of.

The invention is set out in more detail with respect to the drawings, in which
- Fig. 1: shows curves explaining sensor signals in dependence on the time in a situation where no unpleasant odours occur, and
- Fig. 2: shows such signals in case that such unpleasant odours occur, and
- Fig. 3: is a flow chart for explaining a simple embodiment of the inventive method.

In a dishwasher as an example for a household appliance, two sensors applying the identical physical working principle are placed: A first sensor is a metal oxide semi-conductor sensor (MOS sensor) responsive to volatile organic compounds. A second sensor is equally a MOS sensor, but is responsive to hydrogene or to nitrogene oxide(s).

The household appliance and the sensors are not shown in the figures. The dishwasher may be of any known kind and shape. Usually, the dishwasher comprises a housing (not shown in the figures), in which not only elements to place dishes and cutlery thereon are provided, but wherein equally said two sensors are provided.

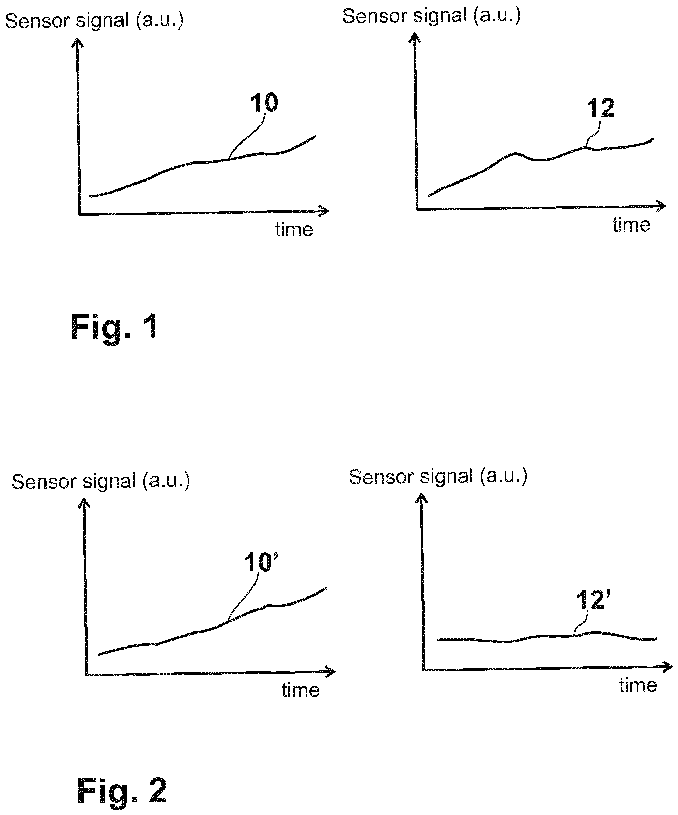

Fig. 1 and 2 show typical curves of the signals output by the sensors (measurement or sensor signal, in arbitrary units).

Fig. 1 shows two curves 10 and 12 under normal conditions without occurrence of unpleasant odours due to a decomposition of organic material. The curve 10 represents the output of the first MOS sensor and the curve 12 represents the output of the second MOS sensor. As can be gathered from these figures, the MOS sensors both show a varying sensor signal, the so-called unstable base line. This means that absolute measurement signals are not an indicator of present gas or odour concentrations. The instability of the base line such as curve 10 is a typical and unavoidable characteristic on the measuring principle in a MOS sensor. An increase in the measurement signal of the first MOS sensor can be caused not only by increased concentrations of volatile organic compounds, but also by changing external conditions of measurement (such as in particular a temperature or a humidity). These drifts cannot be distinguished from slow changes in the sample gas concentration, as it is typically associated with the slow onset and decay of slow development of volatile organic compounds.

The second MOS sensor provides information which is independent of volatile organic compounds, but the outputting of measurement signals is based on the same physical or presently more precisely, physico-chemical principle of measurement. Here, a change in the external measurement conditions will lead to the same signal changes. From fig. 1 one can in particular gather that a drift of the base line 10 of the first MOS sensor has to be detected as a mere drift without increase of the amount of volatile organic compounds in case the measurement values provided by the measurement/sensor signal according to curve 12 is undergoing the same drift.

Fig. 2 now shows the situation in which there is indeed a decomposition of organic material: Here, curve 10' shows an increase in the value of measurement signal output by the first MOS sensor. At the same time, no increase in the sensor signal output by the second MOS sensor can be observed, see curve 12'. (There might be a slight increase in the sensor signal output by the second sensor due to a drift, whilst there is a strong increase in the value of the measurement signal output by the first MOS sensor due to the volatile organic compounds).

Thus, when observing both of these curves at the same time, one can more reliably determine whether or not there are unpleasant odours present.

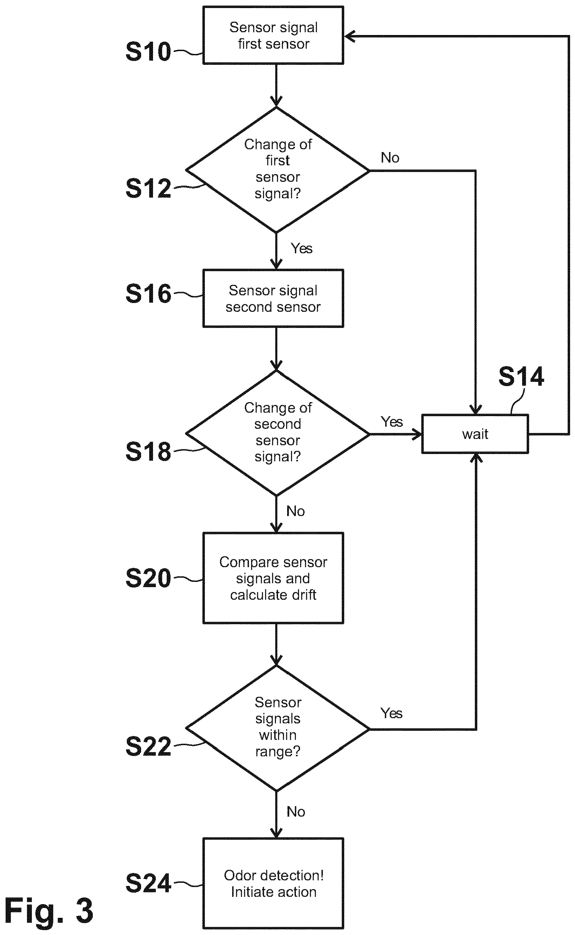

A method of the kind as described hereinafter with regard to fig. 3 is used:
In step S10, one receives a sensor signal from a first sensor.
In step S12, a first pre-determined criterion is applied: Does a change in the first sensor signal occur? If this is not the case, the method goes over to step S14, in which it is waited.

Following step S14, the method newly starts with step S10.

In case that in step S12 a change of the first sensor signal is determined according to a pre-determined criterion which for example includes that starting from an initial value, in a pre-determined time period, the signal height has increased by a pre-determined amount, the method goes over to step S16: In step S16, the sensor signal from the second sensor is received.

Following thereafter in step S18, a second pre-determined criterion is applied: Here, it is determined whether or not the second sensor signal has changed. If this is the case, once again, step S14 (waiting) is performed prior to returning to step S10.

In case that the second pre-determined criterion is not fulfilled which includes for example that a threshold is exceeded, the method continues with step S20: In step S20 the sensor signals are compared, and the drift is in total calculated. Thereafter, in step S22, it is determined whether or not the sensor signals are within a pre-determined range. If so, it is continued with step S14 prior to returning to step S10.

If the sensor signals are not within a pre-determined range, step S24 is the next step: It is now (according to the two pre-determined criteria) clear that an unpleasant odour has been detected, and an action is initiated such as activating a cleaning unit in the dishwasher.

It is to be noted that steps S20 and S22 may be omitted. For example, the determination whether or not the sensor signals are within a range as in step S22 may take place as part of the first and second pre-determined criteria already in complementing steps S12 and S28, respectively.
Whilst with regard to fig. 3, it has been explained that two different pre-determined criteria are applied, the method can be simplified by combining steps S12 and S18 to calculating the ratio of the measurement values provided by the first and provided by the second measurement signals. If the calculated ratio is compared to a threshold value, the same result can be achieved as it is achieved by performing steps S12 and S18: If the first sensor signal increases and the second sensor signal does not increase in a comparable manner (i.e. does not increase at all or increases less), then, the ratio of the first measurement values provided by the first sensor signal to the second measurement values provided by the second sensor signal will increase beyond a pre-determined threshold, that thus being an indication of the occurrence of volatile organic compounds.

## Claims

1. A method of operating a household appliance, in particular a dishwasher, in dependence on the presence of odours due to decomposition of organic material in said household appliance, said method comprising the steps of:
- obtaining (S10) first measurement signals (10, 10') by a first sensor responsive to a first group comprised of at least one first chemical substance which is produced upon decomposition of organic material,
- obtaining (S16) second measurement signals (12, 12') by a second sensor responsive to a second group comprised of at least one second chemical substance which neither is produced upon decomposition of organic material, nor is a detergent or included in a detergent used in said household appliance,
wherein both the first and the second sensors are responsive to the environmental conditions in a comparable manner,
- determining (S12, S18) whether or not the value of a pre-determined function calculated both on the basis of the measurement values provided by the first measurement signals (10, 10') and the measurement values provided by the second measurement signals (12, 12') is higher than a pre-determined threshold value or whether or not the value of a pre-determined function calculated both on the basis of the measurement values provided by the first measurement signals and the measurement values provided by the second measurement signals (12, 12') is lower than a pre-determined threshold value, and if so,
- outputting (S24) a signal indicating the presence of odours due to decomposition of organic material and/or a signal activating a unit in said household appliance acting to reduce such odours.

2. The method according to claim 1, wherein said pre-determined function is a ratio of the measurement values provided by the first or the second measurement signals (10, 10') versus the measurement values provided by the second or first measurement signals (12, 12') respectively.

3. The method of any of claims 1 or 2, wherein a signal is output that activates a cleaning unit in the household appliance, and wherein after the completion of a cleaning step, the measurement signals are obtained and stored for determining the predetermined threshold value.

4. The method according to any of the preceding claims, wherein the physical principles, chemical principles or physico-chemical principles, of how the first and second measurement signals are obtained by the first and second sensor are identical.

5. The method according to any of the preceding claims, wherein the first sensor is a metal oxide semi-conductor sensor, which is in particular responsive to volatile organic compounds.

6. The method according to any of the preceding claims, wherein said second sensor is a metal oxide semiconductor sensor, which is in particular responsive to hydrogene or nitrogene oxide.

7. A household appliance, in particular a dishwasher, comprising two sensors from which a first sensor is responsive to a first group comprised of at least one first chemical substance which is produced upon decomposition of organic material and a second sensor is responsive to a second group comprised of at least one second chemical substance which neither is produced upon decomposition of organic material, nor is a detergent or included in a detergent used in said household appliance, a cleaning unit acting to remove sources of odours in the household appliance, and a control unit receiving measurement signals (10, 10', 12, 12') for the first and second sensors and outputting control signals to said cleaning unit, wherein both of the first and the second sensors are responsive to the environmental conditions in a comparable manner, **characterized in that** said control unit is adapted to use signals of both the first and second sensors in a method according to any of the preceding claims when determining whether or not to output an activating control signal to said cleaning unit.

8. The household appliance according to claim 7, wherein both sensors obtain respective measurement signals by using identical physical principles, chemical principles or physico-chemical principles.

9. The household appliance according to claim 7 or 8, wherein both sensors are metal oxide semi-conductor sensors.

10. The household appliance according to claim 9, wherein a first metal oxide semi-conductor sensor is responsive to volatile organic compounds.

11. The household appliance according to claim 9 or 10, wherein a second metal oxide semi-conductor sensor is responsive to hydrogene and/or nitrogene oxide or nitrogene oxides.

12. The household appliance according to any of claims 7 to 11, wherein the control unit is adapted to continuously or repeatedly receive measurement signals (10, 10¹) from the first sensor and to determine whether or not a first pre-determined criterion is fulfilled, wherein in case the first pre-determined criterion is fulfilled, the control unit receives measurement signals (12, 12') from the second sensor and determines whether or not a second pre-determined criterion is fulfilled.

13. The household appliance according to any of claims 7 to 12, comprising a housing in which the first and second sensors are arranged and/or a sensor housing for each of the first and second sensors.

## Patentansprüche

1. Verfahren zum Betreiben eines Haushaltsgeräts, insbesondere eines Geschirrspülers, in Abhängigkeit von dem Vorhandensein von Gerüchen aufgrund einer Zersetzung von organischem Material in dem Haushaltsgerät, wobei das Verfahren die folgenden Schritte umfasst:
- Erhalten (S10) erster Messsignale (10, 10') durch einen ersten Sensor, der auf eine erste Gruppe anspricht, die aus wenigstens einer ersten chemischen Substanz besteht, die bei einer Zersetzung von organischem Material gebildet wird,
- Erhalten (S16) zweiter Messsignale (12, 12') durch einen zweiten Sensor, der auf eine zweite Gruppe anspricht, die aus wenigstens einer zweiten chemischen Substanz besteht, die weder bei einer Zersetzung von organischem Material gebildet wird, noch ein Reinigungsmittel ist oder in einem Reinigungsmittel, das in dem Haushaltsgerät verwendet wird, enthalten ist, wobei der erste und der zweite Sensor auf die Umgebungsbedingungen in einer vergleichbaren Weise ansprechen,
- Feststellen (S12, S18), ob der Wert einer vorgegebenen Funktion, der auf der Basis der Messwerte, die durch die ersten Messsignale (10, 10') bereitgestellt werden, und der Messwerte, die durch die zweiten Messsignale (12, 12') bereitgestellt werden, berechnet wird, höher als ein vorgegebener Schwellenwert ist oder nicht, oder ob der Wert einer vorgegebenen Funktion, der auf der Basis der Messwerte, die durch die ersten Messsignale bereitgestellt werden, und der Messwerte, die durch die zweiten Messsignale (12, 12') bereitgestellt werden, berechnet wird, niedriger als ein vorgegebener Schwellenwert ist oder nicht, und falls dies zutrifft,
- Ausgeben (S24) eines Signals, das das Vorhandensein von Gerüchen aufgrund einer Zersetzung von organischem Material anzeigt, und/oder eines Signals, das eine Einheit in dem Haushaltsgerät aktiviert, die aktiv wird, um derartige Gerüche zu reduzieren.

2. Verfahren nach Anspruch 1, wobei die vorgegebene Funktion ein Verhältnis der Messwerte, die durch die ersten oder die zweiten Messsignale (10, 10') bereitgestellt werden, zu den Messwerten ist, die durch die zweiten bzw. die ersten Messsignale (12, 12') bereitgestellt werden.

3. Verfahren nach einem der Ansprüche 1 oder 2, wobei ein Signal ausgegeben wird, das eine Reinigungseinheit in dem Haushaltgerät aktiviert, und wobei nach dem Beenden eines Reinigungsschritts die Messsignale erhalten und gespeichert werden, um den vorgegebenen Schwellenwert zu bestimmen.

4. Verfahren nach einem der vorhergehenden Ansprüche, wobei die physikalischen Prinzipien, die chemischen Prinzipien oder die physikalischchemischen Prinzipien, auf welche Weise die ersten und zweiten Messsignale durch den ersten und den zweiten Sensor erhalten werden, identisch sind.

5. Verfahren nach einem der vorhergehenden Ansprüche, wobei der erste Sensor ein Metalloxid-Halbleitersensor ist, der insbesondere auf flüchtige organische Stoffe anspricht.

6. Verfahren nach einem der vorhergehenden Ansprüche, wobei der zweite Sensor ein Metalloxid-Halbleitersensor ist, der insbesondere auf Wasserstoff oder Stickoxid anspricht.

7. Haushaltsgerät, insbesondere ein Geschirrspüler, der zwei Sensoren, wovon ein erster Sensor auf eine erste Gruppe anspricht, die aus wenigstens einer ersten chemischen Substanz besteht, die bei einer Zersetzung von organischem Material gebildet wird, und wovon ein zweiter Sensor auf eine zweite Gruppe anspricht, die aus wenigstens einer zweiten chemischen Substanz besteht, die weder bei einer Zersetzung von organischem Material gebildet wird, noch ein Reinigungsmittel ist oder in einem Reinigungsmittel, das in dem Haushaltsgerät verwendet wird, enthalten ist, eine Reinigungseinheit, die aktiv wird, um Quellen von Gerüchen in dem Haushaltsgerät zu entfernen, und eine Steuereinheit, die Messsignale (10, 10', 12, 12') des ersten und des zweiten Sensors empfängt und Steuersignale an die Reinigungseinheit ausgibt, umfasst, wobei der erste und der zweite Sensor auf die Umgebungsbedingungen auf vergleichbare Weise ansprechen,
**dadurch gekennzeichnet, dass** die Steuereinheit ausgelegt ist, bei der Feststellung, ob ein Steuersignal zur Aktivierung an die Reinigungseinheit auszugeben ist, Signale des ersten und des zweiten Sensors in einem Verfahren nach einem der vorhergehenden Ansprüche zu verwenden.

8. Haushaltsgerät nach Anspruch 7, wobei beide Sensoren entsprechende Messsignale erfassen, indem sie identische physikalische Prinzipien, chemische Prinzipien oder physikalisch-chemische Prinzipien nutzen.

9. Haushaltsgerät nach Anspruch 7 oder 8, wobei beide Sensoren Metalloxid-Halbleitersensoren sind.

10. Haushaltsgerät nach Anspruch 9, wobei ein erster Metalloxid-Halbleitersensor auf flüchtige organische Stoffe anspricht.

11. Haushaltsgerät nach Anspruch 9 oder 10, wobei ein zweiter Metalloxid-Halbleitersensor auf Wasserstoff und/oder Stickoxid oder auf Stickoxide anspricht.

12. Haushaltsgerät nach einem der Ansprüche 7 bis 11, wobei die Steuereinheit ausgelegt ist, kontinuierlich oder wiederholt Messsignale (10, 10') von dem ersten Sensor zu erhalten und festzustellen, ob ein erstes vorgegebenes Kriterium erfüllt ist oder nicht, wobei die Steuereinheit in dem Fall, in dem das erste vorgegebene Kriterium erfüllt ist, Messsignale (12, 12') von dem zweiten Sensor erhält und feststellt, ob ein zweites vorgegebenes Kriterium erfüllt ist oder nicht.

13. Haushaltsgerät nach einem der Ansprüche 7 bis 12, das ein Gehäuse, in dem der erste und der zweite Sensor angeordnet sind, und/oder ein Sensorgehäuse jeweils für den ersten und den zweiten Sensor umfasst.

## Revendications

1. Une Méthode de fonctionnement d'un appareil ménager, en particulier d'un lave-vaisselle, en fonction de la présence d'odeurs dues à une décomposition de matière organique dans ledit appareil ménager, ladite méthode comprenant les étapes de :
- obtenir (S10) des premiers signaux de mesure (10, 10') par le biais d'un premier capteur réagissant à un premier groupe constitué d'au moins une première substance chimique qui est produite lors de la décomposition de matière organique ;
- obtenir (S16) des seconds signaux de mesure (12, 12') par le biais d'un second capteur réagissant à un second groupe constitué d'au moins une seconde substance chimique qui n'est ni produite lors de la décomposition de matière organique, ni détergente ou incluse dans un détergent utilisé dans ledit appareil ménager ;
dans laquelle le premier capteur et le second capteur réagissent tous deux aux conditions environnementales d'une manière comparable ;
- déterminer (S12, S18) si la valeur d'une fonction prédéterminée calculée à la fois sur la base des valeurs de mesure fournies par les premiers signaux de mesure (10, 10') et sur la base des valeurs de mesure fournies par les seconds signaux de mesure (12, 12') est supérieure ou non à une valeur de seuil prédéterminée, ou si la valeur d'une fonction prédéterminée calculée à la fois sur la base des valeurs de mesure fournies par les premiers signaux de mesure et sur la base des valeurs de mesure fournies par les seconds signaux de mesure (12, 12') est inférieure ou non à une valeur de seuil prédéterminée, et si oui ;
- fournir en sortie (S24) un signal indiquant la présence d'odeurs dues à une décomposition de matière organique, et/ou un signal activant une unité, dans ledit appareil ménager, agissant de manière à réduire de telles odeurs.

2. La méthode selon la revendication 1, dans laquelle ladite fonction prédéterminée est un rapport entre les valeurs de mesure fournies par les premiers signaux de mesure ou les seconds signaux de mesure (10, 10') et les valeurs de mesure fournies par les seconds signaux de mesure ou les premiers signaux de mesure (12, 12') respectivement.

3. La méthode selon l'une quelconque des revendications 1 et 2, dans laquelle un signal est fourni en sortie qui active une unité de nettoyage dans l'appareil ménager, et dans laquelle, à l'issue d'une étape de nettoyage, les signaux de mesure sont obtenus et stockés en vue de déterminer la valeur de seuil prédéterminée.

4. La méthode selon l'une quelconque des revendications précédentes, dans laquelle les principes physiques, les principes chimiques ou les principes physico-chimiques concernant la manière dont les premiers et seconds signaux de mesure sont obtenus par les premier et second capteurs sont identiques.

5. La méthode selon l'une quelconque des revendications précédentes, dans laquelle le premier capteur est un capteur à semi-conducteur à oxyde de métal qui réagit en particulier aux composés organiques volatils.

6. La méthode selon l'une quelconque des revendications précédentes, dans laquelle ledit second capteur est un capteur à semi-conducteur à oxyde de métal qui réagit en particulier à l'oxyde d'azote ou d'hydrogène.

7. Un appareil ménager, en particulier un lave-vaisselle, comprenant deux capteurs parmi lesquels un premier capteur réagit à un premier groupe constitué d'au moins une première substance chimique qui est produite lors d'une décomposition de matière organique, et un second capteur réagit à un second groupe constitué d'au moins une seconde substance chimique qui n'est ni produite lors d'une décomposition de matière organique, ni détergente ou incluse dans un détergent utilisé dans l'appareil ménager, une unité de nettoyage agissant de manière à éliminer des sources d'odeurs dans l'appareil ménager, et une unité de commande recevant des signaux de mesure (10, 10', 12, 12') pour les premier et second capteurs et fournissant en sortie des signaux de commande à ladite unité de nettoyage ;
dans lequel le premier capteur et le second capteur réagissent tous deux aux conditions environnementales d'une manière comparable, **caractérisé en ce que** ladite unité de commande est apte à utiliser des signaux à la fois des premier et second capteurs dans le cadre d'un procédé selon l'une quelconque des revendications précédentes lorsqu'il s'agit de déterminer s'il convient de fournir en sortie ou non un signal de commande d'activation à ladite unité de nettoyage.

8. L'appareil ménager selon la revendication 7, dans lequel les deux capteurs obtiennent des signaux de mesure respectifs en utilisant des principes physiques, des principes chimiques, ou des principes physico-chimiques identiques.

9. L'appareil ménager selon la revendication 7 ou 8, dans lequel les deux capteurs sont des capteurs à semi-conducteur à oxyde de métal.

10. L'appareil ménager selon la revendication 9, dans lequel un premier capteur à semi-conducteur à oxyde de métal réagit aux composés organiques volatils.

11. L'appareil ménager selon la revendication 9 ou 10, dans lequel un second capteur à semi-conducteur à oxyde de métal réagit à l'oxyde d'hydrogène et/ou à l'oxyde d'azote ou aux oxydes d'azote.

12. L'appareil ménager selon l'une quelconque des revendications 7 à 11, dans lequel l'unité de commande est apte à recevoir, en continu ou de manière répétée, des signaux de mesure (10, 10') en provenance du premier capteur, et à déterminer si un premier critère prédéterminé est rempli ou non, dans lequel, lorsque le premier critère prédéterminé est rempli, l'unité de commande reçoit des signaux de mesure (12, 12') en provenance du second capteur et détermine si un second critère prédéterminé est rempli ou non.

13. L'appareil ménager selon l'une quelconque des revendications 7 à 12, comprenant un boîtier dans lequel sont agencés les premier et second capteurs et/ou un boîtier de capteur pour le premier capteur et pour le second capteur.
